# EUROPEAN PATENT APPLICATION

(11) **EP 2 554 063 A1**
(43) Date of publication of application: **06.02.2013**
(21) Application number: 11176708.3
(22) Date of filing: 05.08.2011
(51) Int. Cl.: A41D 19/015, D04B 1/28, A61F 13/10

(54) **Ergonomic knitted glove for the support and/or compression of a hand during compression therapy**

(71) Applicant: Tricolast N.V., 9800 Deinze (BE)
(72) Inventor: Oriol, Sylvain, 1860 Aigle (CH)
(74) Representative: Brants, Johan P.E.

(57) **Abstract**

The current invention concerns an ergonomic compression glove and manufacturing method thereof, comprising out of back, palm and finger stalls for the small, ring, middle, index finger and thumb and consisting essentially out of a knitted stretchable fabric and closure seams, whereby said knitted stretchable fabric is subdivided in sections for assembling said back, palm and finger stalls and whereby at least one of the said sections for finger stalls is partially unattached at the lateral side from said section assembling back and palm.

## Description

### TECHNICAL FIELD

The invention pertains to the technical field of knitted garments for compression therapy, more specifically a knitted glove to support and/or compress a hand.

### BACKGROUND

Compression gloves are used in particular to prevent or treat edemas, varicose veins, venous insufficiency, tendinitis, arthritis and after vein surgery. In order for the glove to be affective, that is apply a well-defined amount of pressure to the wearer's hand, the glove needs to mimic the anatomy of the wearer's hand as closely as possible, as to obtain a good fit. The glove should closely resemble the size and shape of the wearer's hand. This is not easily done as the hand has a complicated shape and exhibits many contours and different segments. An important feature thereby is the position of the finger stalls with regards to the back and palm of the hand. For instance, the location of the base of each finger stall at the level of the palm will differ for each finger. Another important feature to note is the width and circumference of the finger stalls. Therefore, in order to obtain a good fit of the glove, with a high degree of comfort for the wearer, while simultaneously applying enough pressure to the hand, the compression glove should be designed with close resemblance to the actual anatomy of the wearer's hand.

Compression gloves are primarily manufactured as knitted garments, due to their good elastic properties. In the prior art, the gloves are generally manufactured as a flat formed body of the actual hand shape consisting out of various sections, that are separately produced on a flatbed knitting machine. On such a flatbed knitting machine, it is relatively easy to also manufacture contours having very complicated shapes using narrowing and knit widening with exact needle placement, and by using spacing techniques. However, the individual pieces manufactured on a flatbed knitting machine must be subsequently sewn together and contain closure seams. Moreover, the compression gloves in prior art are often foreseen from a fourchette, which is a strip or shaped piece of tissue used for the interfinger-regions of the glove.

US 6 554 783 describes a compression glove which comprises of several sections which are sewn together. The glove is designed after fitting the wearer's hand to templates and choosing the most appropriate size of the glove. The interfinger region is formed by a fourchette, placed between the sections that will form the interior and exterior of the finger stalls. Therefore, each finger stall will finally comprise 4 lateral closure seams in the longitudinal direction of the finger stalls. This increases the production costs of the article and results in the presence of seams that can negatively affect wearing comfort due to the formation of pressure points. In addition, seams are also often the very places in a knitted garment that can partially tear when stress is exerted on the knitted garment. This can negatively affect the service life and appearance of the knitted glove as a whole.

US 2 006 002 139 0 discloses a knitted garment such as a compression glove manufactured by a flatbed knitting machine and which is essentially seamless. However, it does not disclose a glove which is tailored entirely to the anatomy wearer's hand. It does not disclose specific methods to ensure that the glove fits the hand in an ergonomic way, for instance by obtaining precise measurements, in order to achieve an optimally fitting glove. A glove not accustomed to the wearer's hand will result in a loss of the desired pressure, as well as create potential discomfort for the wearer, as the glove does not entirely mimic the anatomy of the hand in reality. Certain movements, such as grip force on objects for instance might become hampered when wearing this type of glove.

The current invention aims to provide a compression glove and manufacturing method thereof whereby the number of the lateral seams, longitudinal to the finger stalls is kept to a minimal, while still providing an ergonomic glove that mimics closely the characteristics and anatomy of the wearer's hand. Furthermore, the glove according to the current invention will assure an optimal control of the compression applied on each finger stall. Therefore, the compression glove of the current invention will provide an optimal support and compression to the wearer's hand, as well as ensure the long-term quality of the glove

### SUMMARY OF THE INVENTION

In one aspect, the present invention discloses an ergonomic compression glove, comprising out of back, palm and finger stalls for the small, ring, middle, index finger and thumb and consisting essentially out of a knitted stretchable fabric and closure seams, whereby said knitted stretchable fabric is subdivided in sections for assembling said back, palm and finger stalls and whereby at least one of the said sections for finger stalls comprises attached and unattached regions to said section assembling back and palm.

In another aspect, the present invention discloses a method to manufacture said ergonomic compression glove by knitting a stretchable fabric which is designed by obtaining precise parameters of the wearer's hand, and whereby the said knitted stretchable fabric is folded along its fold-line and whereby the sections meant to assemble the fingers of the glove stalls are stitched together with maximal 2 lateral closure seams longitudinal to said finger stall.

### DESCRIPTION OF FIGURES

**Figures 1 a-d** depict a compression glove according to the prior art, whereby the compression glove is composed out of separately knitted sections stitched together to form the compression glove.
**Figures 2 a-e** depict an exemplary plan of a knitted fabric used to assemble a preferred embodiment of the current invention.
**Figure 3** depicts an exemplary of the compression glove according to the current invention.
**Figure 4** depicts the specific parameters of the hand to be measured in order to design the knitted fabric.

### DETAILED DESCRIPTION OF THE INVENTION

The present invention concerns a knitted compression glove and a manufacturing method thereof whereby the lateral closure seams longitudinal to the finger stalls are kept to a minimal, while still ensuring an optimal ergonomic fit of the glove.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.
The recitation of numerical ranges by endpoints includes all numbers and fractions subsumed within that range, as well as the recited endpoints.
The expression "% by weight" (weight percent), here and throughout the description unless otherwise defined, refers to the relative weight of the respective component based on the overall weight of the formulation.
The term "exterior" of the fingers relates to the particular side of the finger stalls situated at the back portion of the hand.
The term "interior" of the finger relates to the particular side of the finger situated at the palm of the hand.
The term "extensibility" refers to the increase in length or width of a material when it is stretched, as well as its capacity to return to its normal or pre-stretched shape and size when released.
The term "interfinger region" relates to the lateral region of the finger, which is not part of the back of the hand, or the palm of the hand.

In a first aspect, the invention provides for an ergonomic compression glove as described in claim 1, which comprises out of a palm and back portion and 6 finger stalls, for respectively thumb, index, middle, ring and small finger. The said compression glove is designed in such a manner as to provide an ergonomic glove, providing an optimal fit and pressure to the wearer's hand, as well as to provide a rigid and strong glove, with excellent longevity.

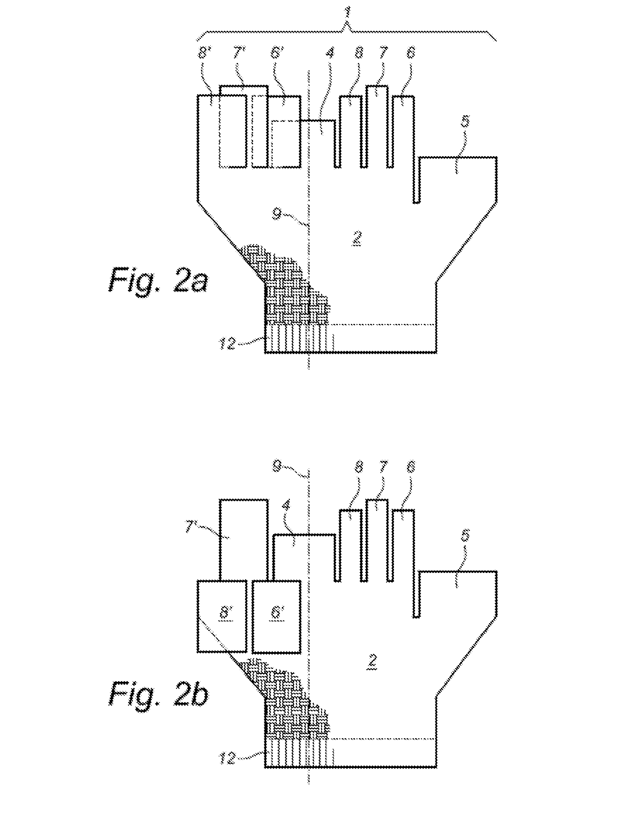

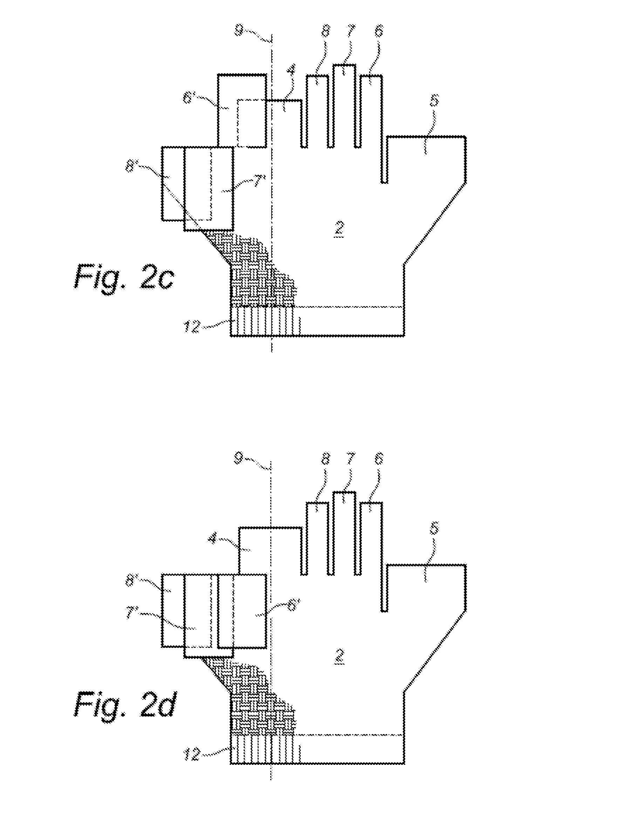

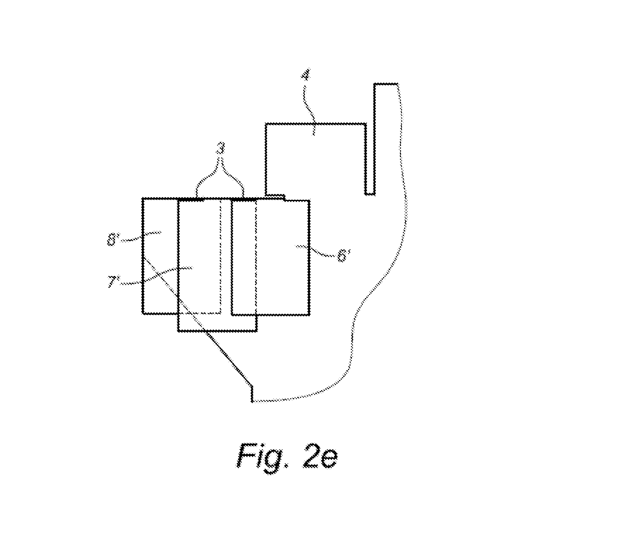

The current invention therefor discloses a glove consisting essentially out of a knitted stretchable fabric as exemplified in figures 2a-e and closure seams, whereby the lateral closure seams longitudinal to the finger stalls are kept to a minimum. In a preferred embodiment, the glove of the current invention comprises a maximum of 2 said lateral closure seams per finger stall. In a most preferred embodiment, the small finger stall and thumb stall comprise only 1 lateral closure seam longitudinal to the finger stall.

In a second aspect, the said knitted stretchable fabric is subdivided in sections which will form the back and palm (2) and the finger stalls (1) of the ergonomic compression glove according to the current invention.

In a preferred embodiment, the said sections for the finger stalls (1) will furthermore be subdivided into sections assembling the interior and exterior of the fingers, as well as the interfinger regions. In a preferred embodiment, the said sections assembling interior, exterior and interfinger regions of each finger stall will be separate or connected entities, depending on the nature of the finger stall to be formed. In another preferred embodiment, the said sections for the finger stalls are attached to the section forming back and palm (2) of the hand. In a more preferred embodiment, at least one of the said sections of the finger stalls comprise attached and unattached regions (3) to said sections for back and palm of hand, and comprises loose flanges with regards to the back and palm sections. The said unattached regions (3) are located at the lateral side of the sections for the finger stalls. These unattached regions allow to vary the width of the sections of the finger stalls, as well as to broaden them, without having to take the width of the section for the back and palm of the hand into account or vary the width of the latter. In a most preferred embodiment, at least one of the said interfinger regions of the compression glove will be formed by the said unattached regions (3) and the latter will have a width equal to the said interfinger regions of the corresponding finger stall. In a further most preferred embodiment, the two interfinger regions of the small finger and index finger stall will be formed by respectively one finger section which comprises only one laterally unattached region to said back and palm sections at one side, while the other side is still attached to said palm and back sections. In contrast, the two interfinger regions of the middle and ring finger stall are both formed by finger stall sections which are on both lateral sides unattached to back and palm section of the glove. In a most preferred embodiment, the said sections for the interfinger regions do not exist as separate entities; they are always interconnected to either said sections assembling the interior regions of the said finger stalls, or the said sections assembling the exterior regions of the finger stalls or connected to both. For instance, in a preferred embodiment, the small finger stall will be assembled by only one finger stall section (4) which will assemble the three subdivisions of the finger stall, said interior, exterior and interfinger regions. The latter is equally the case for the section assembling the thumb stall (5). The remaining finger stalls for the said ring (6, 6'), middle (7, 7') and index finger (8, 8') will each be assembled by at least two finger stall sections.

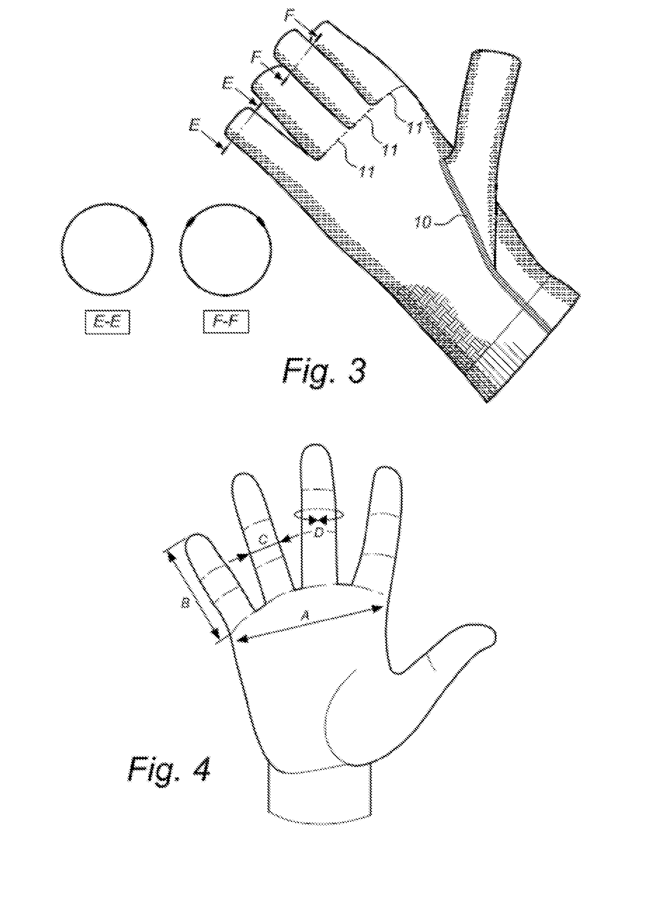

In one embodiment, the said sections for the finger stalls comprise a width of at least 6 meshes. In another embodiment, certain parameters of the wearer's hand will be measured prior to knitting the ergonomic compression glove according to current invention. By designing the compression glove of the current invention based on the obtained parameters, an optimal fit of the glove will be ensured. In a more preferred embodiment, one of the measured parameters is the width A of the palm of said wearer's hand (see figure 4). The said width of the palm A will be measured form the left lateral side to the right lateral side of the hand, just above the implantation of the thumb. Other measured parameters include the length of each finger stall B, the width of each finger stall C and the circumference of each finger stall D. The said parameters may be obtained by manually measuring the hand or automatically by a scanning method. In a most preferred embodiment, the width of the said interfinger region is calculated by the difference between the width A of the palm of the wearer's hand and the sum of the circumferences D of index, ring, middle and small finger, divided by 6.

In another most preferred embodiment, at least one of the said sections for the finger stalls has a width of 20 to 30%, more preferably 25% of the circumference of the corresponding finger of the wearer's hand. More preferentially, 3 of the said sections for the finger stalls comprise a width of 20 to 30% of the circumference of the corresponding finger. Furthermore, at least one of the said sections for the finger stalls has a width of 70% to 80%, more preferably 75% of the circumference of the corresponding finger of the said wearer's hand. More preferentially, 3 of the said sections for the finger stalls comprise a width of 70 to 80% of the circumference of the corresponding finger.

The actual glove according to the current is acquired by folding the said knitted fabric along its fold-line (9) and stitching closure seams along the edges of said folded knitted fabric. In a preferred embodiment, the said fold-line (9) of the knitted fabric lies at the section assembling the said small finger stall (4). The said closure seams comprise lateral closure seams along the longitudinal axis of the finger stalls, transverse closure seams, at the bases of each finger stall, in between two finger stalls, a palm closure seam (10) and a lateral seam to join back and palm portion of the glove. The yarn to be used for the seams can be any yarn readily known in prior art that can be used for seams, more specifically the said yarn for seams can be chosen from the group of cotton, nylon, polyester, polyamide, polypropylene.

The ergonomic compression glove according to the current invention comprises a minimum of lateral closure seams longitudinal to the finger stalls (see figure 3; E-E; F-F). In a preferred embodiment, each finger stall of the said ergonomic compression glove comprises maximal 2 lateral closure seams in the longitudinal direction of said finger stalls. In a most preferred embodiment, the said finger stalls of the small finger and thumb comprise maximal 1 lateral closure seam in the longitudinal direction of said finger stall (E-E).

Compression gloves to be used in the medical field have a compression range between 15 mm Hg and 50 mm Hg. Most mild lymphedema patients with hand swelling require compression in the 20-30 mm Hg range, or for more severe lymphedema in the 30-40 mm Hg range. Currently, flat knit compression glove technology is utilized for higher compression levels for moderate to severe lymphedema.

In order to accomplish the goals of the compression glove of the present invention, it has been found that the fabric must be such as to be stretchable in all directions. Hence, in a preferred embodiment, its fully relaxed state should be slightly smaller than the wearer's hand, but upon receiving the said wearer's hand, it should easily stretch simultaneously in all directions to receive the hand of the wearer and have sufficient elasticity to continuously apply pressure to the hand and finger stalls of the wearer once the glove is in place. The knitted fabric preferentially consists out of a short-stretch material. Short-stretch material with a compression range of 15-100% maximal extensibility is desired, with the preferred embodiment lying in the 30-60% range, but may also consist of inelastic or medium to long stretch fabrics. Extensibility refers to the increase in length or width of a material when it is stretched, as well as its capacity to return to its normal or pre-stretched shape and size when released. For those known in the art, the extensibility of a material is often referred to as the elasticity of the material. The purpose of short-stretch compression is that the product can be applied at or near maximal stretch. The said knitted stretchable fabric can be manufactured using knitting threads and weft threads with elasticity between 1% and 400%, and a linear density of 44 to 3,000 dtex. The elasticity and linear densities of the knitting threads and weft threads can differ. The fabric is preferably woven from synthetic fibres which are capable of meeting the above referred to conditions. The preferred fibres are a composite of polyamide fibres and polyurethanes fibres, also classified generally as spandex fibres. The polyamide fibres such as nylon 6, 6 or nylon 6 melt spun into a continuous filament yarn is highly oriented and has a tenacity of 6 to 9 grams per denier, has a high extensibility and an unusually low modulus at low strength. Its resilience characteristics are good and it has great strength and ability to absorb energy. Nylon 6, 6 is capable of being blended with other fibres on the woolen, worsted and cotton systems of spinning. By blending the nylon 6, 6 with other fibres such as the spandex fibres, it imparts an amazing resistance to wear and greatly reduces felting shrinkage. The most preferable of the spandex fibres for use in the present invention is segmented polyurethane manufactured by Dupont and sold under the trademark Lycra. Lycra has an ultimate extension of about 600% and a reasonably good tenacity of 0.6 to 0.8 grams per denier. Lycra is superior to other elastomers such as rubber and has resistance to sunlight, abrasion, oxidation, oils and chemicals. It has a good flex life and is elastically reversible. When nylon 6, 6 and spandex, such as Lycra, are combined in a ratio of 80% to 20%, in accordance with the preferred embodiment of the present invention, an excellent elastomeric fabric is provided which accomplishes all of the requirements and goals of the compression glove constructed in accordance with the principles of the present invention.

The glove according to the present invention may be a complete glove, comprising closed finger stalls, with rounded, straight or pointed finger tips or may be provided under the form of a gauntlet with thumb stall depending upon the precise application and demands of the wearer.

In another aspect, the current invention provides for a manufacturing method of an ergonomic compression glove according to claim 11.

In one embodiment of the invention, the first step in the manufacturing of the said ergonomic compression glove involves knitting stretchable fabric comprising sections for back and palm of the hand, and finger stalls as exemplified in figures 2a-e. As previously mentioned, the knitted stretchable fabric comprises a fold-line (9) at the finger stall of the small finger. The said knitted stretchable fabric will be folded along said fold-line and subsequently the edges will be joined together by stitching closure seams. The plan of the knitted stretchable fabric will be carefully designed, based upon the obtained parameter of the wearer's hand (see figure 4).

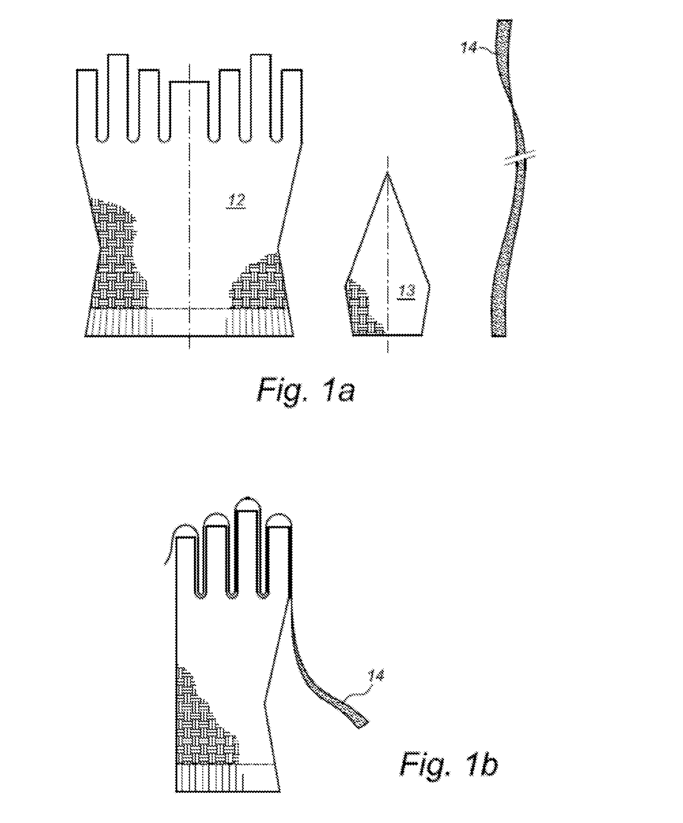

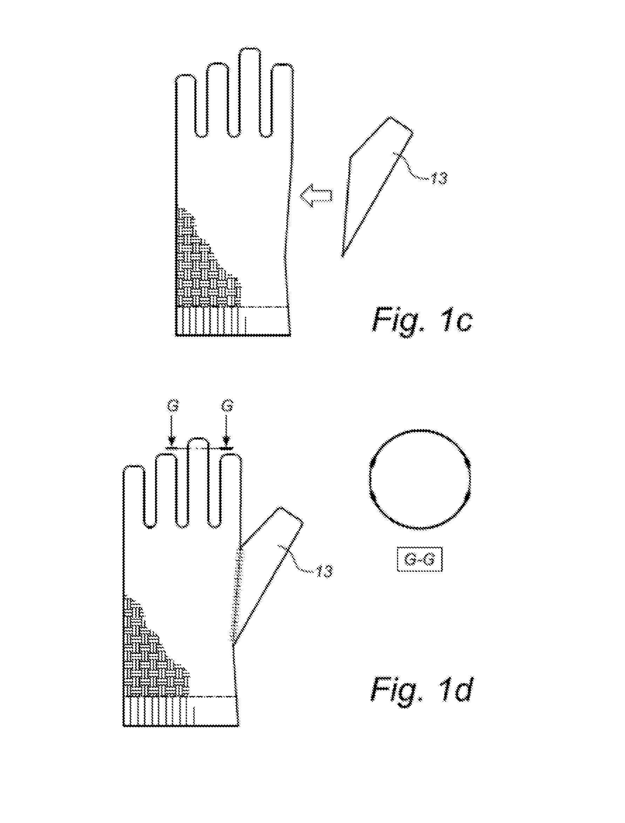

Figures 1a-d show a plan of a knitted stretchable fabric to manufacture a compression glove according to the prior art. The design of fabric shows a precise symmetry, with the axis of symmetry at the section of the small finger (12) and with identical sections intended to assemble the finger stalls on each side of the symmetry axis. The section for the thumb stall (13) is knitted as a separate section (14). A fourchette of a certain width (14) will be positioned between the finger stalls and stitched thereon (see figures 1a-b) in order to form the interfinger regions. In order to obtain a full glove of the fabric sections, the said fabric will be folded along its symmetry axis and both the fourchette and section for the thumb stall will be stitched to said fabric with closure seams (see figures 1c-d). Stitching the fourchette to the finger stalls will subsequently lead to the presence of 4 lateral closure seams (G-G) per finger stall (see figure 1d). These closure seams are generally seen to be the weak points of the glove where tears and ruptures can appear after certain amount of time. These seams are equally the area where the applied pressure or compression on the hand is difficult to control. Therefore, the current invention discloses a knitted fabric as basis for the glove of the current invention, which aims to limit the said lateral closure seams of the finger stalls. Figures 2a-d show an exemplary schematic overview of the said stretchable knitted fabric according to the current invention. The said fabric does not show a symmetry as the glove bases known from the prior art. Furthermore, the section intended for assembling the thumb (5) is integrated in the said fabric, and does not have to be attached in later stages, such as known in the prior art. Each finger stall will be assembled by a maximum of two finger stall sections whereby sections assembling the interfinger regions of the finger stalls are equally foreseen in the said fabric.

Preferentially, the stretched fabric is knitted on a flatbed knitting machine according to a precise scheme of steps (see figures 2a-d):
- knitting a first section (2), intended to assemble back and palm of the glove;
- knitting a second section (4) positioned at the centre of the said first section, intended to assemble the finger stall for the small finger;
- creating an unattached region (3) between said finger stall section and back and palm section by eliminating part of the meshes at one lateral side of the base of said second section (4);
- knitting a third until sixth section (8, 7, 6), intended to respectively assemble finger stall of the ring, middle and index finger;
- knitting a seventh (6') and eighth section (7'), intended to assemble the said finger stall of respectively the ring finger and middle finger;
- creating unattached regions (3) between said finger stall section and back and palm section by eliminating part of the meshes at both lateral sides of the base of said seventh (6') and eighth section (7');
- knitting a ninth section (8'), intended to assemble the finger stall of the index finger;
- creating an unattached region (3) between said finger stall section and back and palm section by eliminating part of the meshes at one lateral side of the base of said ninth section (8');
- knitting a tenth section (5), intended to assemble the said finger stall for the thumb.

Said unattached regions (3) obtained by eliminated meshes on both or either one lateral side of said sections for finger stalls have the width of the interfinger region of the corresponding finger stall.

When arriving at the base of each knitted finger stall, situated at the said section for back and palm, the height of the finger stall basis can be corrected to correlate exactly with the basis (11) of the of the fingers of the wearer's hand. This is achieved by increasing or decreasing the number of meshes by the use of shaping or transfer techniques.

In one embodiment, the section for the thumb stall can be knitted prior to knitting the sections for the other remaining finger stalls. In another embodiment, the said section for the finger stalls can be knitted prior to knitting the said section for the thumb stall.

As mentioned previously, the ergonomic compression glove according to the current invention is adapted exactly to the anatomy of the wearer's hand and aims to achieve a high comfort for the wearer. This can be for instance achieved by knitting ribs in the area of the glove welt, which results in high elasticity and prevents said welt from rolling up. When desired or depending on the application, variations in mesh size, selected weave, chosen knitting threads, material thickness can be applied.

While the current invention has been illustrated and described herein as an ergonomic compression glove for the support and/or compression of a hand, it is not intended to be limited to the details shown, since various modifications and structural changes may be made without departing in any way from the spirit of the present invention.

## Claims

1. An ergonomic compression glove, comprising out of back, palm and finger stalls for the small, ring, middle, index finger and thumb and consisting essentially out of a knitted stretchable fabric and closure seams, whereby said knitted stretchable fabric is subdivided in sections for assembling said back, palm and finger stalls and whereby at least one of the said sections for finger stalls comprises attached and unattached regions to said section assembling back and palm.

2. The ergonomic compression glove according to claim 1, whereby said sections for the finger stalls comprises sections to assemble the interior, exterior and interfinger region of said finger stalls.

3. An ergonomic glove according to claim 2, whereby at least one of said the interfinger-regions of the finger stalls are comprised of the said regions unattached from back and palm sections.

4. The ergonomic compression glove according to claim 3, whereby the width of said interfinger regions are calculated by the difference between the width of the palm of the wearer's hand and the sum of the circumference of each finger, divided by 6.

5. The ergonomic compression glove according to claim 4, whereby the fold-line of said knitted stretchable fabric lies at the section of the finger stall for the small finger.

6. The ergonomic compression glove according to claim 5 whereby each finger stall of said compression glove comprises maximal 2 lateral closure seams in the longitudinal direction of said each finger stall.

7. The ergonomic compression glove according to claim 6, whereby the finger stall of the small finger and the thumb of said glove comprise maximum 1 lateral closure seam in the longitudinal direction of said finger stalls.

8. The ergonomic compression glove according to claim 7, whereby said sections for the finger stalls comprise a width of at least 6 meshes.

9. The ergonomic compression glove according to claim 8, whereby said at least one of the said sections for the finger stalls has a width of 20 to 30%, more preferably 25% of the circumference of the corresponding finger of the wearer's hand.

10. The ergonomic compression glove according to claim 9, whereby at least one of the said sections for the finger stalls has a width of 70% to 80%, more preferably 75% of the circumference of the corresponding finger of the said wearer's hand.

11. A method for manufacturing a compression glove comprising:
- knitting a stretchable fabric whereby plan of said stretchable fabric is designed based upon precise parameters of said wearer's hand.
- folding a knitted stretched fabric along its said fold-line and
- stitching said the sections for the fingers stalls together with maximal 2 lateral closure seams longitudinal to said finger stall.

12. A method for manufacturing an ergonomic compression glove according to claim 11, whereby said stretched fabric is knitted according to the following steps:
- knitting a first section (2), intended to assemble back and palm of the glove;
- knitting a second section (4) positioned at the centre of the said first section, intended to assemble the finger stall for the small finger;
- creating an unattached region (3) between said finger stall section and back and palm section by eliminating part of the meshes at one lateral side of the base of said second section (4);
- knitting a third until sixth section (8, 7, 6), intended to respectively assemble finger stall of the ring, middle and index finger;
- knitting a seventh (6') and eighth section (7'), intended to assemble the said finger stall of respectively the ring finger and middle finger;
- creating unattached regions (3) between said finger stall section and back and palm section by eliminating part of the meshes at both lateral sides of the base of said seventh (6') and eighth section (7');
- knitting a ninth section (8'), intended to assemble the finger stall of the index finger;
- creating an unattached region (3) between said finger stall section and back and palm section by eliminating part of the meshes at one lateral side of the base of said ninth section (8');
- knitting a tenth section (5), intended to assemble the said finger stall for the thumb.

13. A method for manufacturing an ergonomic compression glove according to claim 12, whereby the width of the said unattached region of the said sections equals the width of said interfinger region.

14. A method for manufacturing an ergonomic compression glove according to claim 13, whereby the height of the basis of each said section for the finger stalls is corrected in correlation to the basis of the fingers of the wearer's hand.

15. A method for manufacturing an ergonomic compression glove according to claim 14, whereby the said section for the finger stall of the thumb is knitted prior or after knitting the said sections of the finger stalls of the remaining fingers.
